(19) Europäisches Patentamt
European Patent Office
Office européen des brevets

(11) EP 3 967 371 A1

(12) EUROPEAN PATENT APPLICATION
published in accordance with Art. 153(4) EPC

(43) Date of publication:
16.03.2022 Bulletin 2022/11

(21) Application number: 20805686.1

(22) Date of filing: 28.04.2020

(51) International Patent Classification (IPC):
A61Q 5/00 (2006.01) A61Q 5/04 (2006.01)
A61Q 5/06 (2006.01) A61Q 5/12 (2006.01)
A61K 8/362 (2006.01) A61K 8/365 (2006.01)
A61K 8/46 (2006.01)

(52) Cooperative Patent Classification (CPC):
A61K 8/362; A61K 8/365; A61K 8/46; A61Q 5/00; A61Q 5/04; A61Q 5/06; A61Q 5/12

(86) International application number:
PCT/JP2020/018109

(87) International publication number:
WO 2020/230624 (19.11.2020 Gazette 2020/47)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 10.05.2019 JP 2019090210
28.02.2020 JP 2020033808

(71) Applicant: Kao Corporation
Tokyo 103-8210 (JP)

(72) Inventors:
- NIWANO, Yu
Tokyo 131-8501 (JP)
- ITAYA, Miki
Tokyo 131-8501 (JP)

(74) Representative: Hoffmann Eitle
Patent- und Rechtsanwälte PartmbB
Arabellastraße 30
81925 München (DE)

# (54) HAIR TREATMENT METHOD

(57) A hair treatment method comprising steps [A-1], [B], and [C], wherein the method is carried out in the order of [A-1] → [B] → [C], or in the order of [B] → [A-1] → [C]:
step [A-1]: a step of applying a first hair treatment agent comprising an organic acid having a solubility in 100 mL of water at 20°C of 50 g or less or a salt of the organic acid, as component (A1), to hair, and rinsing the hair;
step [B]: a step of applying a second hair treatment agent comprising a carboxylic acid having an inorganic value of 250 or more and 450 or less and an organic value of 50 or more and 250 or less or a salt of the carboxylic acid, as component (B), to the hair; and
step [C]: a step of applying a third hair treatment agent comprising an aromatic sulfonic acid having a molecular weight of 300 or less or a salt thereof, as component (C), to the hair.

EP 3 967 371 A1

## Description

### Field of the Invention

The present invention relates to a hair treatment method.

### Background of the Invention

Curls artificially given to hair by techniques such as permanent treatment have problems in that the curl direction is partially reversed as the days go on, so that not only a beautiful appearance is impaired but also the hair is easily entangled with fingers. However, there has conventionally been no prior art intended to solve these problems.

### Summary of the Invention

In a first aspect, the present invention provides a hair treatment method comprising the following steps [A-1], [B], and [C], wherein the method is carried out in the order of step [A-1] → step [B] → step [C], or in the order of step [B] → step [A-1] → step [C]:

step [A-1]: a step of applying a first hair treatment agent comprising an organic acid having a solubility in 100 mL of water at 20°C of 50 g or less or a salt of the organic acid, as component (A1), to hair, and rinsing the hair;
step [B]: a step of applying a second hair treatment agent comprising a carboxylic acid having an inorganic value of 250 or more and 450 or less and an organic value of 50 or more and 250 or less or a salt of the carboxylic acid, as component (B), to the hair; and
step [C]: a step of applying a third hair treatment agent comprising an aromatic sulfonic acid having a molecular weight of 300 or less or a salt thereof, as component (C), to the hair.

In a second aspect, the present invention also provides a hair treatment method comprising the following steps [A-2], [B], and [C], wherein the method is carried out in the order of step [A-2] → step [B] → step [C], or in the order of step [B] → step [A-2] → step [C]:

step [A-2]: a step of applying a first hair treatment agent comprising an organic acid being more hydrophobic than a carboxylic acid of component (B) or a salt of the organic acid, as component (A2), to hair;
step [B]: a step of applying a second hair treatment agent comprising a carboxylic acid having an inorganic value of 250 or more and 450 or less and an organic value of 50 or more and 250 or less or a salt of the carboxylic acid, as the component (B), to the hair; and
step [C]: a step of applying a third hair treatment agent comprising an aromatic sulfonic acid having a molecular weight of 300 or less or a salt thereof, as component (C), to the hair.

### Detailed Description of the Invention

The present invention relates to a hair treatment method which uniformly aligns the direction of naturally frizzy hair and curls given to hair by permanent treatment and the like to recover beauty appearance and which can make the hair less tangled and improve the manageability of curls.

As a result of intensive studies, the present inventors have found that, not only in the curls artificially given by a technique such as permanent treatment but also in the naturally frizzy hair, calcium ions in the water are accumulated in the hair during daily repeated hair washing, so that the direction of the curls is partially reversed to make a random shape, which impairs the beauty and makes the hair easily tangled.

As a result of further intensive studies, the present inventors have found that the treatment of hair with a hydrophobic organic acid, a specific carboxylic acid, and an aromatic sulfonic acid in a predetermined order allows the calcium ions accumulated in the hair to flow out, and the carboxylic acid and the aromatic sulfonic acid modify the inside of the hair, so that a beautiful curl shape in which the curl phase of the hair after treatment is uniformly aligned is obtained and the hair is less tangled, thereby completing the invention.

The hair treatment method of the present invention uniformly aligns the direction of naturally frizzy hair and curls given to hair by permanent treatment and the like to recover beauty appearance and makes the hair less tangled, and can improve the manageability of curls.

- First Hair Treatment Agent (Used in Step [A-1] and Step [A-2]) [Component (A1)]

The first hair treatment agent used in step [A-1] contains an organic acid having a solubility in 100 mL of water at 20°C of 50 g or less or a salt of the organic acid, as the component (A1). The solubility in the component (A1) refers to the solubility of the organic acid. That is, also when the component (A1) is an organic acid salt, it means the solubility of the free acid thereof. From the viewpoint of allowing calcium in the hair to flow out and allowing the component (A1) itself to remain in the hair to modify the hair, the solubility of the component (A1) in 100 mL of water at 20°C is 50 g or less, preferably 40 g or less, more preferably 30 g or less, further preferably 25 g or less, further preferably 15 g or less, and further preferably 10 g or less. Specific examples of the organic acid in the component (A1) include succinic acid (the solubility is 5.8 g), tartaric acid (the solubility is 20.6 g), mandelic acid (the solubility is 15.5 g), and phenyllactic acid (the solubility is 5.3 g) . From the viewpoint of the effect of flowing calcium out of the hair, succinic acid and tartaric acid are preferable, and succinic acid is more preferable.

[Component (A2)]

The first hair treatment agent used in step [A-2] contains an organic acid being more hydrophobic than the carboxylic acid of the component (B) or a salt of the organic acid, as the component (A2). As used herein, being more hydrophobic than the carboxylic acid of the component (B) means that the solubility in 100 mL of water at 20°C is lower than that of the carboxylic acid of the component (B). Here, the solubility to be compared is the solubility of the organic acid also when the component (A2) is an organic acid salt, and is the solubility of the carboxylic acid also when the component (B) is a carboxylic acid salt. Specific examples of the component (A2) include succinic acid (the solubility is 5.8 g) and tartaric acid (the solubility is 20.6 g) when the component (B) is malic acid (the solubility is 55.8 g) or lactic acid (the solubility is 876 g), and succinic acid is preferable from the viewpoint of the effect of flowing calcium out of the hair.
From the viewpoint of improving the formulation stability, the content of the component (A1) or (A2) in the first hair treatment agent is preferably 15% by mass or less, more preferably 12% by mass or less, further preferably 8% by mass or less, and further preferably 6% by mass or less, and from the viewpoint of improving the spiral rate, uniformity of the curl, and detanglability of the curl of the hair after treatment, it is preferably 0.5% by mass or more, more preferably 1.5% by mass or more, further preferably 2.5% by mass or more, further preferably 3.5% by mass or more, and further preferably 4.5% by mass or more.

[Water]

The first hair treatment agent preferably uses water as a solvent, from the viewpoint of ease of blending and penetration into the hair.
From the viewpoint of improving the penetration into the hair of the component (A1) or the component (A2), the pH of the first hair treatment agent at 25°C is preferably 6.5 or less, and more preferably 4.5 or less, and from the viewpoint of alleviating the irritation to the skin and the scalp skin, it is preferably 2 or more, and more preferably 3 or more.
In addition to the component (A1) and the component (A2), the first hair treatment agent may appropriately contain other components such as an anionic surfactant, a nonionic surfactant, a zwitterionic surfactant, a cationic polymer, and a solvent.
Examples of the form of the first hair treatment agent include shampoos, cleansing conditioners, and preshower treatments.
- Second Hair Treatment Agent (Used in Step [B])

The second hair treatment agent contains a carboxylic acid having an inorganic value of 250 or more and 450 or less and an organic value of 50 or more and 250 or less or a salt of the carboxylic acid, as the component (B), and used in step [B]. When the first hair treatment agent is the agent used in step [A-1], an aspect in which the second hair treatment agent is the same composition as this first hair treatment agent is included, but from the viewpoint of improving the outflow of calcium ions and the penetration of the aromatic sulfonic acid, the second hair treatment agent is preferably a composition different from the first hair treatment agent.

[Component (B)]

The component (B) is a carboxylic acid having an inorganic value of 250 or more and 450 or less and an organic value of 50 or more and 250 or less or a salt of the carboxylic acid. The inorganic value and the organic value in the component (B) refer to the inorganic value and the organic value of the carboxylic acid. That is, in the case of a carboxylic acid salt, they mean the inorganic value and the organic value for the free acid. From the viewpoint of efficiently penetrating the component (B) itself to the hair and improving the amount of penetration and the penetration rate of other components,

the inorganic value is preferably 260 or more, and more preferably 265 or more, and preferably 420 or less, and more preferably 400 or less. From the same viewpoint, the organic value is preferably 60 or more, and more preferably 80 or more, and preferably 200 or less, and more preferably 180 or less.

The inorganic value and the organic value are the approaches based on an organic conception diagram, and use the values calculated based on "Formulation Design with Organic Conception Diagram" (Yamori; Fragrance Journal, 1989(4), P29 to 38).

Examples of such a carboxylic acid include carboxylic acids selected from the group consisting of the carboxylic acid of the following formula (B-1), malic acid, succinic acid, and lactic acid.

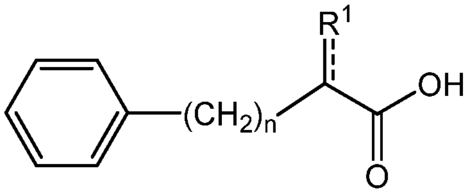

(B-1)

wherein $R^1$ represents a hydrogen atom, an oxygen atom, or a hydroxy group; the dashed line represents a double bond when $R^1$ is an oxygen atom; and n represents an integer of 0 or more and 3 or less; and a portion of the phenyl group and the methylene chain may be substituted with a hydroxy group.

Examples of the carboxylic acid of formula (B-1) include mandelic acid and phenyllactic acid.

Specific inorganic values and organic values of these carboxylic acids are mandelic acid (265, 160), phenyllactic acid (265, 180), malic acid (400, 80), succinic acid (300, 80), and lactic acid (250, 60). The numerical values in the parentheses indicate the inorganic value and the organic value, respectively.

From the viewpoint of efficiently penetrating the component (B) itself into the hair and improving the penetration into the hair of the component (C), the component (B) is preferably at least one selected from the group consisting of mandelic acid, phenyllactic acid, malic acid, succinic acid, and salts thereof. Among them, at least one selected from the group consisting of malic acid, mandelic acid, phenyllactic acid, and salts thereof is more preferable, and at least one selected from the group consisting of malic acid, phenyllactic acid, and salts thereof is more preferable. From the viewpoint of attaining the effects in a short time, malic acid and a salt thereof are preferable, and further, from the viewpoint of ease of blending, malic acid is preferable.

Examples of the salts of the above carboxylic acids include a sodium salt, a potassium salt, a lithium salt, an aluminum salt, an ammonium salt ($NH^{4+}$), an organic quaternary ammonium salt, and an arginine salt.

These carboxylic acids or salts thereof may be used alone or in combination of two or more. From the viewpoint of improving the penetration of the component (C), the content of the component (B) in the second hair treatment agent is preferably 1% by mass or more, more preferably 2.5% by mass or more, further preferably 5% by mass or more, further preferably 7.5% by mass or more, and further preferably 10% by mass or more, and from the viewpoint of alleviating the irritation to the skin, it is preferably 25% by mass or less, more preferably 20% by mass or less, and further preferably 15% by mass or less.

[Component (D)]

The second hair treatment agent can further contain at least one compound selected from the group consisting of (d1) a glycylglycine derivative of formula (1) or a salt thereof, (d2) a polyglycerin having an average degree of polymerization of 2 or more and 6 or less, and (d3) an alkyl glyceryl ether in which the alkyl group has 6 or more and 40 or less carbon atoms, as the component (D).

The component (d1) is a glycylglycine derivative of formula (1) or a salt thereof.

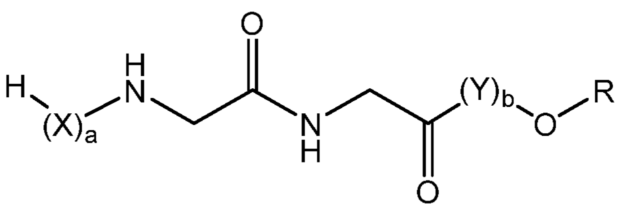

(1)

wherein X represents an optionally hydroxy group-substituted divalent hydrocarbon group having 1 to 4 carbon atoms, or an amino acid residue;

Y represents an amino acid residue, or a divalent group represented by Formula (2):

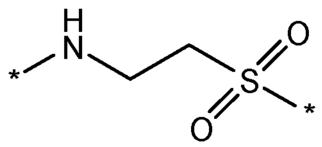

(2)

wherein, -* represents a bond which bonds to an adjacent carbonyl group or oxygen atom;

R represents a hydrogen atom or an optionally hydroxy group-substituted monovalent hydrocarbon group having 1 to 4 carbon atoms; and
a and b represent 0 or 1, provided that, when a and b are simultaneously 1, X is not an amino acid residue.

The glycylglycine derivative of formula (1) or the salt thereof may be in a free form, or may be in the form of amphoteric ion.

Examples of the salt of the glycylglycine derivative include inorganic acid salts such as hydrochloride and sulfate; organic acid salts such as lactate; an ammonium salt ($NH^{4+}$); organic ammonium salts such as an alkyl ammonium salt; and alkaline metal salts such as a sodium salt.

In the formula (1), the optionally hydroxy group-substituted divalent hydrocarbon group having 1 to 4 carbon atoms represented by X may be saturated or unsaturated and may be linear or branched, and from the viewpoint of improving the resistance to shampooing of the effects, a divalent saturated hydrocarbon group substituted with a hydroxy group or a divalent saturated hydrocarbon group is preferable among them.

Examples of the divalent hydrocarbon group include a methylene group, an ethylene group, an ethylidene group, a vinylene group, a trimethylene group, an isopropylidene group, a 1-propenylene group, a tetramethylene group, a 2-methyltrimethylene group, a 1-methyltrimethylene group, and a 1-butenylene group.

Examples of the divalent hydrocarbon group substituted with a hydroxy group include a 1-hydroxyethylene group, a 1-hydroxytrimethylene group, a 1,2-dihydroxytrimethylene group, a 1-hydroxytetramethylene group, a 1,2-dihydroxytetramethylene group, a 1,3-dihydroxytetramethylene group, and a 1,2,3-trihydroxytetramethylene group.

In the formula (1), the "amino acid residue" represented by X means a unit amino acid moiety that is to form an oligopeptide, which is obtained by synthesis or is derived from all amino acids present in the living body, and the unit amino acid moiety may be a D form or an L form.

Examples of the amino acid residue represented by X include a basic amino acid residue such as an arginine residue, a lysine residue, and a histidine residue; an aliphatic amino acid residue such as an alanine residue and a glycine residue; an aromatic amino acid residue such as a phenylalanine residue, a tyrosine residue, and a tryptophan residue; an acid amide amino acid residue such as a glutamine residue and an asparagine residue; an acidic amino acid residue such as a glutamic acid residue, aspartic acid residue, and a cysteic acid residue; a hydroxyamino acid residue such as a serine residue and a threonine residue; and a cyclic amino acid residue such as a proline residue, an N-methylproline residue, and a 4-hydroxy proline residue. Among them, from the viewpoint of high affinity with the tissue inside the hair, at least one selected from the group consisting of an arginine residue, an alanine residue, a phenylalanine residue, a glycine residue, a glutamine residue, glutamic acid residue, a serine residue, a proline residue, an N-methylproline residue, and a 4-hydroxy proline residue is preferable.

In the formula (1), examples of the amino acid residue represented Y include the same residues as the above X, but from the viewpoint of high affinity with the tissue inside the hair, Y is preferably at least one selected from the group consisting of an arginine residue, an alanine residue, a glycine residue, a glutamine residue, a glutamic acid residue, a serine residue, a proline residue, a 4-hydroxy proline residue, and a divalent group of the chemical formula (2).

In the formula (1), the optionally hydroxy group-substituted monovalent hydrocarbon group having 1 to 4 carbon atoms represented by R may be saturated or unsaturated, and may be linear or branched. As the monovalent hydrocarbon group, an alkyl group is preferable, and examples thereof include a methyl group, an ethyl group, a propyl group, a butyl group, an isopropyl group, an isobutyl group, an s-butyl group, and a t-butyl group.

As the monovalent hydrocarbon group substituted with a hydroxy group, a hydroxyalkyl group is preferable, and examples thereof include a hydroxymethyl group, a 2-hydroxyethyl group, a 3-hydroxypropyl group, a 4-hydroxybutyl group, a 2,3-dihydroxyethyl group, a 2,3,4-trihydroxybutyl group, and a 2,4-dihydroxybutyl group.

Examples of the glycylglycine derivative suitable in the present invention include a compound of any of formulas (G1) to (G10), and from the viewpoint of high affinity with the tissue inside the hair, penetration into the hair, and improving the persistence of the effects, a compound of any of formulas (G3) to (G10) is more preferable, and a compound of any of formulas (G9) and (G10) (glycylglycylglycine and glycylglycine) is further preferable. These glycylglycine derivatives may be in a free form, may be an amphoteric ion, or may form a salt. These may be used alone or in combination of

two or more.

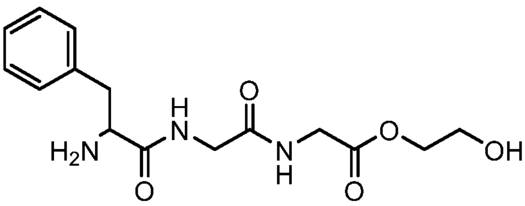

(G1)

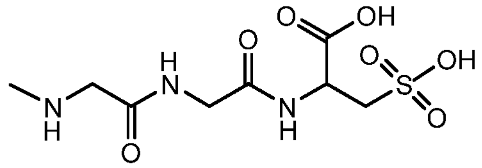

(G2)

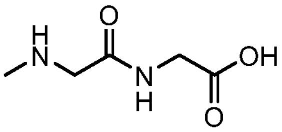

(G3)

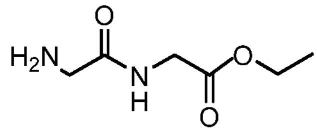

(G4)

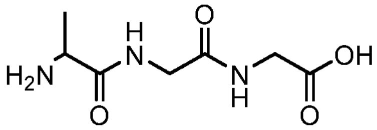

(G5)

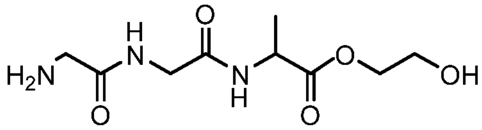

(G6)

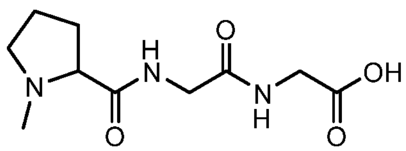

(G7)

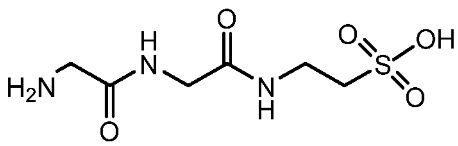

(G8)

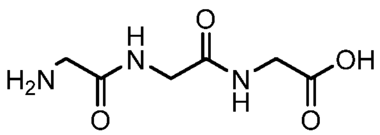

(G9)

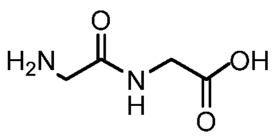

(G10)

Examples of the polyglycerin of the component (d2) having an average degree of polymerization of 2 or more and 6 or less include diglycerin, triglycerin, tetraglycerin, and pentaglycerin, and from the viewpoint of sufficiently penetrating the component into the hair, one having an average degree of polymerization of 2 or more and 4 or less is preferable, and one having an average degree of polymerization of 2.5 or more and 3.5 or less is more preferable.

As the alkyl glyceryl ether of the component (d3) in which the alkyl group has 6 or more and 40 or less carbon atoms, one in which the alkyl group has 8 or more carbon atoms, and further 14 or more carbon atoms is preferable, and 20 or less carbon atoms, and further 18 or less carbon atoms is preferable, from the viewpoint of improving the solubility in water and the penetration into the hair. In addition, this alkyl group is preferably a branched alkyl group. Specific examples thereof include isostearyl glyceryl ether, isodecyl glyceryl ether, and ethylhexyl glyceryl ether.

As the component (D), the components (d1), (d2), and (d3) may be used either alone or in combination of two or more. Among them, the component (d1) is preferably contained as the component (D), from the viewpoint of efficiently improving the persistence of the effects.

When the second hair treatment agent contains both of the components (B) and (D), the aspect of the second hair treatment agent may be a one-part hair treatment agent, or may be a two-part hair treatment agent which is applied to the hair by mixing or sequentially applying a first part containing either one of the component (B) and (D) and a second

part containing another one. As used herein, the content of each component when the second hair treatment agent is a two-part hair treatment agent refers to the content in the total amount of the second hair treatment agent which is the sum of the first part and the second part.

From the viewpoint of improving the persistence of the effects, the content of the component (D) in the second hair treatment agent is preferably 0.5% by mass or more, more preferably 0.8% by mass or more, and further preferably 1.0% by mass or more, and from the viewpoint of formulation stability, it is preferably 10% by mass or less, more preferably 8% by mass or less, and further preferably 6% by mass or less.

[Component (E): Aromatic Alcohol]

The second hair treatment agent may further contain an aromatic alcohol as the component (E), from the viewpoint of promoting the penetration into the hair of the component (B), or the components (B) and (D). Examples of such an aromatic alcohol include benzyl alcohol, cinnamyl alcohol, phenethyl alcohol, p-anisyl alcohol, p-methylbenzyl alcohol, phenoxyethanol, and 2-benzyloxyethanol. Among these, at least one selected from the group consisting of benzyl alcohol and 2-benzyloxyethanol is preferable, and benzyl alcohol is more preferable, from the viewpoint of the compatibility with the component (B).

These aromatic alcohols may be used either alone or in combination of two or more. From the viewpoint of promoting the penetration into the hair of the component (B), or the component (B) and (D), the content of the component (E) in the second hair treatment agent is preferably 0.4% by mass or more, more preferably 1% by mass or more, further preferably 2% by mass or more, and still more preferably 3% by mass or more, and from the same viewpoint, it is preferably 15% by mass or less, more preferably 10% by mass or less, further preferably 8% by mass or less, and still more preferably 6% by mass or less.

In the second hair treatment agent, from the viewpoint of formulation stability, the mass ratio of the component (B) to the component (E), (B)/(E) is preferably 0.5 or more, more preferably 0.75 or more, further preferably 1.0 or more, and still more preferably 1.2 or more, and from the same viewpoint, it is preferably 50 or less, more preferably 20 or less, further preferably 10 or less, still more preferably 5 or less, and still more preferably 2.5 or less.

[Component (G): Organic Solvent]

From the viewpoint of promoting the penetration into the hair of the component (B), or the component (B) and (D), the second hair treatment agent may further contain an organic solvent of the following formula (G-1) as the component (G).

$$R^2\text{- }(OCH_2CH_2)_q\text{-}R^3 \qquad \text{(G-1)}$$

wherein, $R^2$ represents a hydrogen atom or an alkyl group having one or more and 5 or less carbon atoms; $R^3$ represents a hydrogen atom or a hydroxy group; q represents an integer of 0 or more and 5 or less, provided that when q is 0, $R^2$ and $R^3$ are not a hydrogen atom.

Examples of such a component (G) include monohydric alcohols having 1 or more and 4 or less carbon atoms, and ethylene glycol monoalkyl ethers in which R2 is a linear or branched alkyl having 1 or more and 5 or less carbon atoms and q is an integer of 1 or more and 5 or less. Specific examples thereof include ethanol, propanol, isopropanol, ethylene glycol monomethyl ether, ethylene glycol monoethyl ether, ethylene glycol monobutyl ether, diethylene glycol monomethyl ether, diethylene glycol monoethyl ether, and diethylene glycol monobutyl ether. Among these, ethanol is more preferable, from the viewpoint of promoting the penetration into the hair of the component (B).

From the viewpoint of promoting the penetration into the hair of the component (B), or the component (B) and (D), the content of the component (G) in the second hair treatment agent is preferably 5% by mass or more, more preferably 7.5% by mass or more, further preferably 10% by mass or more, and still more preferably 12.5% by mass or more, and from the same viewpoint, it is preferably 35% by mass or less, more preferably 30% by mass or less, further preferably 25% by mass or less, and still more preferably 22% by mass or less.

[Component (F): Thickener]

The second hair treatment agent used in step [B] preferably further contains a thickener as the component (F), from the viewpoint of suppressing the water evaporation rate after application and promoting the penetration into the hair of the component (B), or the component (B) and (D). Examples of the thickener include an anionic thickener, a cationic thickener, and a nonionic thickener.

Specific examples of the anionic thickener include a polyacrylic acid (e.g., Noveon Inc.: Carbopol 941, Carbopol 981), acrylic acid·alkyl methacrylate copolymer (e.g., Noveon Inc.: Carbopol ETD2020), a hydrolysate of a polymer

partially crosslinked with a terminally unsaturated diene compound in a lower alkyl vinyl ether/maleic anhydride copolymer or a monoalkyl ester of the hydrolysate (ASHLAND Inc.: Stabileze 06, Stabileze QM), carrageenan (e.g., MITSUBISHI RAYON CO., LTD.: SOAGEENA LX22, SOAGEENA ML210), xanthan gum (e.g., Sumitomo Dainippon Pharma Co., Ltd.: ECHO GUM T), welan gum (e.g., SANSHO Co., Ltd.: K1C376, K1A96), hydroxypropyl xanthan gum (e.g., Sumitomo Dainippon Pharma Co., Ltd.: Rhaball gum EX), sodium stearoxy PGhydroxyethylcellulose sulfonate, and hydroxyethyl acrylate/sodium acryloyldimethyl taurate copolymer (e.g., SEPPIC Inc.: SIMULGEL NS, SEPINOV EMT10).

Examples of the cationic thickener include natural or semi-synthetic cationic polysaccharides and synthetic polymers having an amino group or an ammonium group on the side chain of polymer chains or containing a diallyl quaternary ammonium salt as a constitutional unit.

Specific examples of the cationic polysaccharides include cationized cellulose derivatives (e.g., Lion Corporation: LEOGARD G, LEOGARD GP; The Dow Chemical Company: UCARE Polymer JR-125, UCARE Polymer JR-400, UCARE Polymer JR-30M, UCARE Polymer LR-400, UCARE Polymer LR-30M; Akzo Nobel N.V.: CELQUAT H-100, CELQUAT L-200), cationized guar gum derivatives (e.g., Solvay S.A.: JAGUAR C-13S, JAGUAR C-17; DSP GOKYO FOOD & CHEMICAL Co., Ltd.: Rhaball gum CG-M, Rhaball gum CG-M7, Rhaball gum CG-M8M), hydroxypropylchitosan (e.g., ICHIMARU PHARCOS Co., Ltd.: Chitofilmer HV-10), and chitosan·dl-pyrrolidone carboxylate (e.g., Union Carbide Corporation: Kytamer PC).

Examples of synthetic cationic polymers having an amino group or an ammonium group on the side chain of polymer chains include synthetic cationic polymers containing trialkylaminoalkyl (meth)acrylate, trialkylaminoalkyl (meth)acrylamide, (meth)acrylamide, vinyl amine, or the like as a constitutional unit, and specific examples thereof include a polymer of methacryloyloxyethylene trimonium chloride (INCI name: polyquaternium-37, e.g., BASF Corporation: Cosmedia ultra gel 300, SALCARE SC95, Sigma 3V: synthalen CR), an (acrylic acid/methyl acrylate/3-methacryloylaminopropyltrimethylammonium chloride) copolymer (INCI name: polyquaternium-47, e.g., The Lubrizol Corporation: Merquat 2201), an (acrylic acid/acrylamide/methylmethacrylamidopropyltrimethylammonium chloride) copolymer (INCI name: polyquaternium-53, e.g., The Lubrizol Corporation: Merquat 2003), a (dimethylacrylamide/ethyltrimonium chloride methacrylate) copolymer (e.g., BASF Corporation: Tinobis CD), and a (vinyl amine/vinyl alcohol) copolymer (e.g., Sekisui Specialty Chemicals: SEVOL ULTALUX AD, Mitsubishi Chemical Corporation: Diafix C-601).

Specific examples of synthetic cationic polymers containing a diallyl quaternary ammonium salt as a constitutional unit include a polymer of diallyl dimethyl ammonium chloride (INCI name: polyquaternium-6, e.g., The Lubrizol Corporation: Merquat 100), a (dimethyldiallylammonium chloride/acrylamide) copolymer (INCI polyquaternium-7, e.g., The Lubrizol Corporation: Merquat 550, Merquat 740), an (acrylic acid/diallyl dimethyl ammonium chloride) copolymer (INCI name: polyquaternium-22, e.g., The Lubrizol Corporation: Merquat 280, Merquat 295), and an (acrylamide/acrylic acid/diallyl dimethyl ammonium chloride) copolymer (INCI name: polyquaternium-39, e.g., The Lubrizol Corporation: Merquat plus 3330, Merquat plus 3331).

Examples of nonionic thickening polymers include natural or semi-synthetic nonionic polysaccharides and synthetic nonionic polymers containing vinyl alcohol or oxyalkylene as a constitutional unit.

Specific examples of natural or semi-synthetic nonionic polysaccharides include water-soluble natural polysaccharides such as starch, guar gum, locust bean gum, and glucomannan; and water-soluble hydroxyalkylated polysaccharides obtained by reacting an alkylene oxide with cellulose, starch, guar gum, locust bean gum, or the like. Specific examples thereof include guar gum (e.g., DSP GOKYO FOOD & CHEMICAL Co., Ltd.: Fiberon S), and pullulan (e.g., HAYASHIBARA CO., LTD.: pullulan PI-20). Examples include hydroxyethylcellulose (e.g., Daicel Finechem Ltd.: SE-850, The Dow Chemical Company: Cellosize HEC QP-52000-H), methyl hydroxyethylcellulose (Akzo Nobel N.V.: STRUCTURE CELL 12000M), hydroxypropylcellulose (e.g., Nippon Soda Co., Ltd.: HPC-H, HPC-M, HPC-L), and hydroxypropyl methylcellulose (e.g., Shin-Etsu Chemical Co., Ltd.: Metolose 60SH-10000).

Specific examples of synthetic nonionic thickening polymers containing vinyl alcohol or oxyalkylene as a constitutional unit include polyvinyl alcohol (e.g., The Nippon Synthetic Chemical Industry Co., Ltd.: GOHSENOL EG-40, GOHSENOL GH-05, GOHSENOL KH-20, GOHSENOL NH-26), highly polymerized polyethylene glycol (e.g., The Dow Chemical Company: POLYOX WSR N-60K, POLYOX WSR301, WSR303), and PEG-240/HDI copolymer bis-decyltetradeceth-20 ether (e.g., ADEKA CORPORATION: Adekanol GT-700).

Among these thickeners, from the viewpoint of suppressing the water evaporation rate after application and promoting the penetration into the hair of the component (B), or the component (B) and (D), natural or semi-synthetic polysaccharides are preferable, at least one selected from the group consisting of xanthan gum, hydroxy xanthan gum, and hydroxyethylcellulose is more preferable, and at least one selected from the group consisting of xanthan gum and hydroxy xanthan gum is more preferable.

The thickener may be used alone or in combination of two or more. The content of the component (F) in the second hair treatment agent is preferably 0.1% by mass or more, more preferably 0.5% by mass or more, and further preferably 1% by mass or more, and preferably 5% by mass or less, and more preferably 3% by mass or less.

[Water]

The second hair treatment agent preferably uses water as a solvent, from the viewpoint of ease of blending and penetration into the hair.

[Component (C): Aromatic Sulfonic Acid or Salt Thereof]

It is preferable that the second hair treatment agent contain neither aromatic sulfonic acid nor salt thereof which are the component (C) contained in the third hair treatment agent used in step [C] described below, i.e., the content be preferably 0% by mass. Even when the second hair treatment agent contains the component (C) (e.g., 0.0001% by mass or more in the composition), the content thereof is preferably less than 3% by mass, more preferably less than 1% by mass, and further preferably less than 0.5% by mass.

The second hair treatment agent may further contain a surfactant. However, from the viewpoint of achieving both the natural application as a formulation and the uniformity of the second hair treatment agent as a preparation, the content of the surfactant in the second hair treatment agent is preferably 2% by mass or less, more preferably 1% by mass or less, further preferably 0.1% by mass or less, further preferably 0.01% by mass or less, and further preferably 0% by mass.

[pH]

From the viewpoint of promoting the penetration into the hair of the aromatic sulfonic acid or a salt thereof as the component (C) in step [C], the pH of the second hair treatment agent is preferably 2 or more, more preferably 2.5 or more, and further preferably 2.8 or more, and preferably 10 or less, more preferably 9 or less, and further preferably 8 or less. From the viewpoint of further reducing damage to the hair and the scalp skin while penetrating a certain amount of the component (B) and the component (C) into the hair, making a feel when the second hair treatment agent is applied to the hair satisfactory, and making the second hair treatment agent easily applicable to the hair, the pH of the second hair treatment agent is more preferably more than 5, and further preferably 6 or more in the above range. On the other hand, from the viewpoint of improving the penetration of the component (B) and component (C), the second hair treatment agent is preferably acidic. In consideration of both viewpoint, the pH of the second hair treatment agent is preferably 7.5 or less, more preferably 7.0 or less, further preferably 6.5 or less, and further preferably 6.0 or less, and preferably 3 or more, and more preferably 4 or more. The pH of the above composition is a value measured with a pH meter under the temperature conditions of 25°C.

When the second hair treatment agent is a two-part hair treatment agent, the pH of the composition containing the component (B) of the first part and the second part may be within any range as long as the pH is within a range typically usable as a hair treatment agent, and is preferably 2 or more, more preferably 3 or more, further preferably 3.5 or more, further preferably 4 or more, and further preferably 5 or more, and preferably 10 or less, more preferably 9 or less, and further preferably 8 or less. From the viewpoint of further reducing damage to the hair and the scalp skin while penetrating a certain amount of the component (B) and the component (C) into the hair, making a feel when the composition is applied to the hair satisfactory, and making the composition easily applicable to the hair, the pH of the above composition is more preferably more than 5, and further preferably 6 or more in the above range. On the other hand, from the viewpoint of improving the penetration of the component (B) and component (C), the above composition is preferably acidic. In consideration of both viewpoint, the pH of the above composition is preferably 7 or less, and more preferably 5 or less, and preferably 3 or more, and more preferably 4 or more. The pH of the above composition is a value measured with a pH meter under the temperature conditions of 25°C.

When the second hair treatment agent is a two-part hair treatment agent, the pH of the composition containing the component (D) of the first part and the second part is preferably 7.5 or less, and more preferably 7.0 or less, from the viewpoint of not inhibiting the penetration into the hair of the aromatic sulfonic acid or a salt thereof as the component (C) in step [C], and is further preferably 6.5 or less, and further preferably 6.0 or less, from the viewpoint of promoting the penetration into the hair of the component (C) in step [C]. From the viewpoint of promoting the penetration into the hair of the aromatic sulfonic acid or a salt thereof as the component (C) in step [C] and suppressing the irritation to the skin, the pH of the above composition is preferably 2 or more, more preferably 2.5 or more, and further preferably 2.8 or more. The pH of the second hair treatment agent is a value measured with a pH meter under the temperature conditions of 25°C.

- Third Hair Treatment Agent (Used in Step [C])

[Component (C): Aromatic Sulfonic Acid Having Molecular Weight of 300 or less or Salt Thereof]

Examples of the aromatic sulfonic acid having a molecular weight of 300 or less or a salt thereof, as the component (C) contained in the third hair treatment agent include naphthalenesulfonic acids, azulenesulfonic acids, benzophenonesulfonic acids, and benzenesulfonic acids.

Examples of the naphthalenesulfonic acids include the compound of the following formula (3).

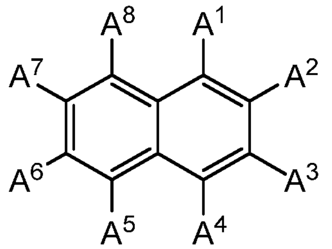

(3)

wherein, one or more of $A^1$ to $A^8$ represent a sulfo group or a salt thereof, and the remaining represents a hydrogen atom, a halogen atom, a hydroxy group, a nitro group, a carboxy group, a lower alkoxycarbonyl group, an alkyl group, an alkenyl group, a lower alkoxy group, a formyl group, an acyl group, an optionally substituted phenylazo group, or -N(R') (R") (R' and R" represent a hydrogen atom, a lower alkyl group, a lower alkenyl group, a phenyl group, a benzyl group, or an acyl group).

Specific examples of the naphthalenesulfonic acids include 1-or 2-naphthalenesulfonic acid (α- or β-naphthalenesulfonic acid), 2,7-naphthalenedisulfonic acid, 1,5-naphthalenedisulfonic acid, 2,6-naphthalenedisulfonic acid, 1-naphthol-2-sulfonic acid, 1-naphthol-4-sulfonic acid, 2-naphthol-6-sulfonic acid, 2-naphthol-7-sulfonic acid, 2,3-dihydroxynaphthalene-6-sulfonic acid, 1,7-dihydroxynaphthalene -3-sulfonic acid, chromotropic acid (4,5-dihydroxynaphthalene -2,7-disulfonic acid), 3,6-dihydroxynaphthalene-2,7-disulfonic acid, S acid (1-amino-8-naphthol-4-sulfonic acid), gamma acid (2-amino-8-naphthol-6-sulfonic acid), J acid (2-amino-5-naphthol-7-sulfonic acid), 1-amino-2-naphthol-4-sulfonic acid, 1-naphthylamine-4-sulfonic acid, Bronner's acid (2-naphthylamine-6-sulfonic acid), Cleves acid (1-naphthylamine-7-sulfonic acid), 2-naphthylamine-1-sulfonic acid, 1-naphthylamine-6-sulfonic acid, 1-naphthylamine-8-sulfonic acid, 2,7-diamino-1-naphthol-3-sulfonic acid, 7,8-diamino-1-naphthol-3-sulfonic acid, a naphthalenesulfonic acid-formalin polycondensate having a molecular weight of 300 or less, 6-methyl-2-naphthalenesulfonic acid, 4-ethyl-1-naphthalenesulfonic acid, 5-isopropyl-1-naphthalenesulfonic acid, 5-butyl-2-naphthalenesulfonic acid, and salts thereof.

Specific examples of the azulenesulfonic acids include guaiazulenesulfonic acid, 1-azulenesulfonic acid, 3-acetyl-7-isopropyl-1-azulenesulfonic acid, 3-(2-hydroxyethyl)-7-isopropyl-1-azulenesulfonic acid, 3-methyl-7-isopropyl-1-azulenesulfonic acid, 7-isopropyl-1-azulenesulfonic acid, 3-phenyl-6-isopropyl-1-azulenesulfonic acid, 1,4-dimethyl-7-isopropyl-2-azulenesulfonic acid, 4-ethoxy-3-ethyl-6-isopropyl-1-azulenesulfonic acid, 1,3-azulenedisulfonic acid, 4,6,8-trimethyl-1,3-azulenedisulfonic acid, 3-formyl-4,6,8-trimethyl-1-azulenesulfonic acid, and salts thereof.

Examples of the benzophenonesulfonic acids include the compound of the following formula (4).

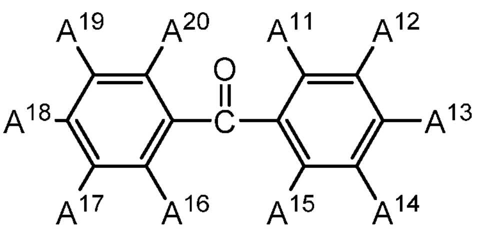

(4)

wherein, one or more of $A^{11}$ to $A^{20}$ represent a sulfo group or a salt thereof, and the remaining represents a hydrogen atom, a halogen atom, a hydroxy group, a carboxy group, an amino group, a lower alkyl group, a lower alkenyl group, a lower alkoxy group, or an acyl group.

Specific examples of the benzophenonesulfonic acids include oxybenzenesulfonic acid, o-chlorobenzophenonesulfonic acid, p-chlorobenzophenonesulfonic acid, 2-hydroxybenzophenonesulfonic acid, 4-hydroxybenzophenonesulfonic acid, 2-aminobenzophenonesulfonic acid, 4-aminobenzophenonesulfonic acid, 2-methylbenzophenonesulfonic acid, 4-methoxybenzophenonesulfonic acid, 4,4'-dimethylbenzophenonesulfonic acid, 4,4'-dimethoxybenzophenonesulfonic acid, and salts thereof.

Examples of the benzenesulfonic acids include the compound of the following formula (5).

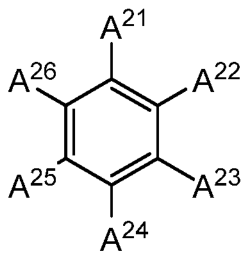

(5)

wherein, one or more of $A^{21}$ to $A^{26}$ represent a sulfo group or a salt thereof, and the remaining represents a hydrogen atom or a lower alkyl group.

Specific examples of the benzenesulfonic acids include benzenesulfonic acid, o-toluenesulfonic acid, p-toluenesulfonic acid, xylenesulfonic acid, cumenesulfonic acid, ethylbenzenesulfonic acid, 2,4,6-trimethylbenzenesulfonic acid, and salts thereof.

Examples of the salts of the above aromatic sulfonic acids include a sodium salt, a potassium salt, a lithium salt, an aluminum salt, an ammonium salt ($NH^{4+}$), and an organic ammonium salt.

From the viewpoint of improving the elasticity in the hair and further uniformizing the hair diameter to make the hair less tangled, the aromatic sulfonic acid or a salt thereof of the component (C) is preferably at least one selected from the group consisting of the naphthalenesulfonic acids of formula (3), the benzophenonesulfonic acids of formula (4), and the benzenesulfonic acids of formula (5), and further, more preferably at least one selected from the group consisting of 2-naphthalenesulfonic acid (β-naphthalenesulfonic acid), 1-naphthalenesulfonic acid (α-naphthalenesulfonic acid), p-toluenesulfonic acid, xylenesulfonic acid, hydroxymethoxybenzophenonesulfonic acid (oxybenzone-5), and salts thereof. Among them, from the above viewpoint, 1- or 2-naphthalenesulfonic acid (α- or β-naphthalenesulfonic acid) or a salt thereof is further preferable.

These aromatic sulfonic acids or salts thereof may be used alone or in combination of two or more. From the viewpoint of promoting the penetration into the hair of the component (C), the content of the component (C) in the third hair treatment agent is preferably 1% by mass or more, more preferably 2.5% by mass or more, further preferably 4% by mass or more, further preferably 5% by mass or more, and further preferably 7% by mass or more, and from the viewpoint of improving the soft touch after hair treatment and the detanglability of the hair, it is preferably 25% by mass or less, more preferably 20% by mass or less, further preferably 15% by mass or less, and further preferably 10% by mass or less.

[Component (F): Thickener]

The third hair treatment agent preferably further contains the above-described thickener as the component (F), from the viewpoint of suppressing the water evaporation rate after application and promoting the penetration into the hair of the component (C). The thickener may be used alone or in combination of two or more. From the viewpoint of promoting the penetration into the hair of the component (C) and giving natural application, the content of the component (F) in the third hair treatment agent is preferably 0.1% by mass or more, more preferably 0.5% by mass or more, and further preferably 1.0% by mass or more, and preferably 5% by mass or less, and more preferably 3% by mass or less.

[Component (E): Aromatic Alcohol]

The third hair treatment agent may further contain an aromatic alcohol as the component (E), from the viewpoint of promoting the penetration into the hair of the component (C). Examples of such an aromatic alcohol include benzyl alcohol, cinnamyl alcohol, phenethyl alcohol, p-anisyl alcohol, p-methylbenzyl alcohol, phenoxyethanol, and 2-benzyloxyethanol. Among these, from the viewpoint of compatibility with the aromatic sulfonic acid, at least one selected from the group consisting of benzyl alcohol and 2-benzyloxyethanol is preferable, and benzyl alcohol is more preferable.

These aromatic alcohols may be used either alone or in combination of two or more. The content of the component (E) in the third hair treatment agent is preferably 1% by mass or more, more preferably 2% by mass or more, and further preferably 4% by mass or more, from the viewpoint of improving the elasticity in the hair and further uniformizing the hair diameter to give beautiful curls, and improving the detanglability of the hair, and is preferably 30% by mass or less, more preferably 20% by mass or less, further preferably 15% by mass or less, and further preferably 10% by mass or less, from the same viewpoint.

In the third hair treatment agent, the mass ratio of the component (C) to the component (E), (C)/(E) is preferably 0.25 or more, more preferably 0.3 or more, further preferably 0.35 or more, and further preferably 0.5 or more, from the viewpoint of formulation stability, and is preferably 2.5 or less, more preferably 2 or less, further preferably 1.8 or less,

further preferably 1 or less, further preferably 0.75, and further preferably 0.7 or less, from the same viewpoint.

[Component (G): Organic Solvent]

The third hair treatment agent may further contain an organic solvent of the following formula (G-1) as the component (G):

$$R^2\text{- }(OCH_2CH_2)_q\text{-}R^3 \qquad (G\text{-}1)$$

wherein, $R^2$ represents a hydrogen atom or an alkyl group having 1 or more and 5 or less carbon atoms; $R^3$ represents a hydrogen atom or a hydroxy group; and q represents an integer of 0 or more and 5 or less, provided that when q is 0, $R^2$ and $R^3$ are not a hydrogen atom.

Examples of such a component (G) include monohydric alcohols having 1 or more and 4 or less carbon atoms, and ethylene glycol monoalkyl ethers in which $R^2$ is a linear or branched alkyl having 1 or more and 5 or less carbon atoms and q is an integer of 1 or more and 5 or less. Specific examples thereof include ethanol, propanol, isopropanol, ethylene glycol monomethyl ether, ethylene glycol monoethyl ether, ethylene glycol monobutyl ether, diethylene glycol monomethyl ether, diethylene glycol monoethyl ether, and diethylene glycol monobutyl ether.

From the viewpoint of improving the spiral rate, uniformity of the curl, and detanglability of the curl of the hair after treatment, the content of the component (G) in the third hair treatment agent is preferably small, and more preferably contains no component (G), i.e., the content is more preferably 0% by mass. Specifically, the content is preferably 3% by mass or less, more preferably 2% by mass or less, further preferably 1% by mass or less, and further preferably 0% by mass.

[Component (H): Polyhydric Alcohol]

The third hair treatment agent may further contain a polyhydric alcohol as the component (H). Examples of the polyhydric alcohol include alcohols having 2 to 20 carbon atoms, specifically, alkylene glycols such as ethylene glycol, diethylene glycol, triethylene glycol, polyethylene glycol, propylene glycol, dipropylene glycol, polypropylene glycol, 1,2-butylene glycol, 1,3-butylene glycol, pentylene glycol, and hexylene glycol; glycerins such as glycerin, and polyglycerin excluding the component (d2); sugar alcohols such as Xylit, Mannit, Galaktit, and Sorbit; and trimethylol ethane, trimethylol propane, and pentaerythritol. Among these, from the viewpoint of improving the spiral rate, uniformity of the curl, and detanglability of the curl of the hair after treatment, alkylene glycols are preferable, and at least one selected from the group consisting of propylene glycol, dipropylene glycol, and polypropylene glycol is preferable.

[Polymeric Surfactant]

The third hair treatment agent may further contain a polymeric surfactant, from the viewpoint of improving the spiral rate, uniformity of the curl, and detanglability of the curl of the hair after treatment. As the polymeric surfactant, a silicone-type surfactant such as an oxazoline-modified silicone such as polysilicone-9, a polyether-modified silicone, and an aminopolyether-modified silicone is preferable.

[Water]

Also, the third hair treatment agent preferably uses water as a solvent, from the viewpoint of sufficiently penetrating the component (C) into the hair.

[pH]

The pH of the third hair treatment agent may be within any range as long as the pH is within a range typically used as a hair treatment agent, and is preferably pH 3 or more and 10 or less, and more preferably 3.5 or more and 8 or less, from the viewpoint of not damaging the hair and the scalp skin. From the viewpoint of modifying the hair to have a high spiral rate after treatment, making a feel when the third hair treatment agent is applied to the hair satisfactory, and making the composition easily applicable to the hair, the pH of the third hair treatment agent is more preferably 5 or more and 8 or less. The pH of the third hair treatment agent is a value obtained by measuring the third hair treatment agent diluted 10 fold with an ion-exchange water with a pH meter under temperature conditions of 25°C.

It is preferable that any of the first to third hair treatment agents substantially contain no further hair reducing agent, from the viewpoint of suppressing chemical damage to the hair. The present invention is characterized in that the deformation of hair is enabled without cutting the S-S bond of protein in the hair, and is a clearly different technique

from a permanent agent in which the deformation of the hair is performed by cutting the S-S bond of protein in the hair using a reducing agent. Examples of the hair reducing agent include thiols such as thioglycolic acid, dithioglycolic acid, cysteine, and acetylcysteine, and bisulfite and salts thereof.

In the present invention, "substantially contain no" refers that the content of the target compound in the hair treatment agent is preferably less than 0.1% by mass, more preferably less than 0.01% by mass, and no target compound is further preferably contained in the hair treatment agent.

- Hair Treatment Method

The first aspect in the hair treatment method of the present invention is carried out by performing the following [A-1], [B], and [C], in the order of step [A-1] → step [B] → step [C], or in the order of step [B] → step [A-1] → step [C].

Step [A-1]: A step of applying the first hair treatment agent to hair and rinsing off the hair
Step [B]: A step of applying the second hair treatment agent to the hair
Step [C]: A step of applying the third hair treatment agent to the hair

The second aspect in the hair treatment method of the present invention includes the following steps [A-2], [B], and [C], and is carried out by performing the steps in the order of step [A-2] → step [B] → step [C], or in the order of step [B] → step [A-2] → step [C].

Step [A-2]: A step of applying the first hair treatment agent to hair
Step [B]: A step of applying the second hair treatment agent to the hair
Step [C]: A step of applying the third hair treatment agent to the hair

From the viewpoint of preventing hair damage, the hair is preferably untangled with hands or a tool such as a comb and a hairbrush, before the first-time step among step [A-1], step [A-2], and step [B]. The hair may be washed or may not be washed before the first-time step, but when a step of washing the hair is included, a commercially available shampoo and the like can be used in washing.

[Step [A-1]: Step of Applying First Hair Treatment Agent to Hair and then Rinsing the Hair]

Step [A-1] in the first aspect is the step of applying the first hair treatment agent to hair and then rinsing the hair, and may be performed at first or may be performed after step [B]. Since the first hair treatment agent contains the organic acid of the component (A1), calcium ions present in the hair can be released out of the hair, resulting in that the effects of the hair treatment caused by the second hair treatment agent and the third hair treatment agent can be more exerted.

[Step [A-2]: Step of Applying First Hair Treatment Agent to the Hair]

Step [A-2] in the second step is the step of applying the first hair treatment agent to the hair, and may be performed at first or may be performed after step [B]. Since the first hair treatment agent contains the organic acid of the component (A2), calcium ions present in the hair can be released out of the hair, resulting in that the effects of the hair treatment caused by the second hair treatment agent and the third hair treatment agent can be more exerted.

The amount of the first hair treatment agent to be applied to the hair in step [A-1] and step [A-2] is such that the bath ratio relative to the mass of the hair (mass of hair treatment agent/mass of hair) is preferably 0.02 or more, more preferably 0.05 or more, and further preferably 0.1 or more, and preferably 2.0 or less, more preferably 1.0 or less, and further preferably 0.5 or less.

To spread the first hair treatment agent over the entire head hair after applying the first hair treatment agent to the hair, a method with hands such as massaging the composition into the hair or hand combing the hair, a method using a tool such as a brush, a comb, and a hairbrush, and a combination of both methods can be used.

Further, in step [A-1] in the first step, the hair is needed to be rinsed after the first hair treatment agent is applied to the hair to release the calcium accumulated in the hair out of the hair, and the hair after rinsing may be dried or may not be dried.

On the other hand, in step [A-2] in the second step, the hair may be rinsed or may not be rinsed after the first hair treatment agent is applied to the hair.

[Step [B]: Step of Applying Second Hair Treatment Agent to the Hair]

Step [B] is the step of applying the second hair treatment agent to the hair, and the step may be performed at first or may be performed after step [A-1] or step [A-2]. Since the second hair treatment agent contains the component (B), the penetration into the hair of the component (C) contained in the third hair treatment agent can be promoted. Further, the second hair treatment agent preferably contains the component (D) in addition to the component (B), which improves the resistance to shampooing of the effects of enhancing the spiral rate, uniformity of the curl, and detanglability of the curl of the hair after treatment.

Specific procedure of step [B] illustrated according to each form of the hair treatment agent is as follows. In the following description about the treatment method of the hair, the simple term "hair treatment agent" refers to the composition actually applied to the hair, and the term also refers to any of a one-part hair treatment agent, a mixture of the first part and second part of a single-application type two-part hair treatment agent, and the first part and second part of a sequential-application type two-part hair treatment agent.

• In the case where the second hair treatment agent is a one-part hair treatment agent

Step [B-a]: A step of applying a one-part hair treatment agent to the hair

Not only the case where the second hair treatment agent contains the components (B) and (D), but also the case where only the component (B) is contained and the component (D) is not contained are included in this type.

• In the case where the second hair treatment agent is a single-application type two-part hair treatment agent

Step [B-b]: A step of mixing the first part and second part of a two-part hair treatment agent, and then applying the mixture to the hair

• In the case where the second hair treatment agent is a sequential-application type two-part hair treatment agent

Step [B-c]: A step of applying the first part of the two-part hair treatment agent to hair, and then applying the second part of the two-part hair treatment agent over the first part application portion of the hair

Matters Common to Step [B-a], Step [B-b], and Step [B-c]

In any of the above cases, the second hair treatment agent may be applied to the dried hair or may be applied to the wet hair, but is preferably applied to the dried hair. The hair to be treated may be the whole head hair or may be a portion thereof.

The amount of the second hair treatment agent applied to the hair in step [B] is such that the bath ratio relative to the mass of the hair (mass of hair treatment agent/mass of hair) is preferably 0.05 or more, more preferably 0.1 or more, and further preferably 0.25 or more, and is preferably 1.5 or less, more preferably 1.25 or less, and further preferably 1.0 or less.

To spread the second hair treatment agent over the entire head hair after applying the second hair treatment agent to the hair, a method with hands such as massaging the composition into the hair or finger combing the hair, a method using a tool such as a brush, a comb, and a hairbrush, and a combination of both methods can be used.

In the Case of Step [B-a] and Step [B-b]

In the case where a one-part hair treatment agent or a single-application type two-part hair treatment agent is used, i.e., in the case of the step [B-a] or step [B-b], the component (B) and the component (D) are simultaneously applied to the hair, and thus, there is no need to provide a standing time between the applications of both components. Thus, these aspects are preferable from the viewpoint of shortening the procedure time.

In the Case of Step [B-c]

In the case where a sequential-application type two-part hair treatment agent is used, the first part is only required to be applied to the hair in accordance with the above-described conditions, and then the second part may be applied to the hair in accordance with the above-described conditions, but it is preferable to apply the second part to the portion where the first part is applied. Either one of the first part and the second part may contain the component (B) and the other may contain the component (D), or vice versa. That is, the first part containing the component (B) may be applied to hair, and then the second part containing the component (D) may be applied over the first part application portion of the hair, or the first part containing the component (D) may be applied to hair, and then the second part

containing the component (B) may be applied over the first part application portion of the hair.

In the case where the sequential type two-part hair treatment agent is used, it is preferable to provide further the following step [b2] after the first part is applied to the hair and before the second part is applied to the hair.

Step [b2]: A step of leaving the hair to stand at 15°C or more and 100°C or less for 15 seconds or more and 60 minutes or less

The temperature for standing in step [b2] is 15°C or more and 100°C or less, preferably 60°C or less, and more preferably 30°C or less. The temperature is preferably 15°C or more and less than 30°C, i.e., room temperature, in terms of convenient treatment without a need for a special device in the hair treatment. On the other hand, from the viewpoint of further shortening the time for leaving the hair to stand, it is possible to stand while warming with a heater and the like, and the temperature in this case is preferably 30°C or more, and more preferably 40°C or more, and 100°C or less, and more preferably 60°C or less.

From the viewpoint of improving the spiral rate, uniformity of the curl, and detanglability of the curl of the hair after treatment, the time for leaving the hair to stand in step [b2] is 15 seconds or more, preferably 30 seconds or more, more preferably 1 minute or more, and preferably 3 minutes or more, and more preferably 5 minutes or more, and 60 minutes or less, preferably 45 minutes or less, and more preferably 30 minutes or less. When standing is performed while heating with a heater and the like as described above, the time for leaving the hair to stand can be further shortened, and the standing time in this case is 15 seconds or more, preferably 30 seconds or more, more preferably 1 minute or more, preferably 3 minutes or more, and more preferably 5 minutes or more, and preferably 30 minutes or less, more preferably 20 minutes or less, and further preferably 15 minutes or less.

In the present invention, the standing time in step [b2] means the time after the first part is applied to the hair and until the next step, i.e., until the step other than the standing step.

Typically, the penetration into the hair of a component is advantageously performed by leaving the hair to stand under heating conditions for a long period of time, but the present invention is excellent in terms of obtaining the advantageous effects of the invention even by standing at room temperature for a short time.

Step [b2] is preferably performed in an environment in which the evaporation of moisture is suppressed. Examples of specific means for suppressing the evaporation of moisture include a method for covering the hair to which the first part is applied with a plastic film, a cap, or the like, and a method for continuously spraying water vapor such as superheated water vapor to the hair.

The first part may be rinsed or may not be rinsed from the hair, after standing and before applying the second part. In the case where the hair is rinsed after standing, the hair may be dried or may not be dried after rinsing and before applying the second part. After rinsing and before applying other compositions, the hair may be untangled by hands or with a tool such as a comb or a hairbrush, or may not be untangled.

After leaving the hair to stand in step [b2], the second part is only required to be applied to the hair in accordance with the conditions.

[Step [b1]: Step of Leaving the Hair to stand at 15°C or more and 100°C or less for 15 Seconds or more and 60 Minutes or less, After Step [B]]

After step [B], in the case where step [B] is the first-time step, step [A-1] or step [A-2] may be performed without standing, and in the case where step [B] is the step performed subsequently to step [A-1] or step [A-2], step [C] may be performed without standing, but the following step [b1] may be performed.

Step [b1]: A step of leaving the hair to stand at 15°C or more and 100°C or less for 15 seconds or more and 60 minutes or less, after step [B]

The temperature for leaving the hair to stand in step [b1] is 15°C or more and 100°C or less, preferably 60°C or less, and more preferably 30°C or less. The temperature is preferably 15°C or more and less than 30°C, i.e., room temperature, in terms of convenient treatment without a need for a special device in the hair treatment. On the other hand, from the viewpoint of shortening the standing time, it is possible to leaving the hair to stand while warming with a heater and the like, and the temperature in this case is preferably 30°C or more, and more preferably 40°C or more, and 100°C or less, and more preferably 60°C or less.

The time for leaving the hair to stand in step [b1] is 15 seconds or more, preferably 30 seconds or more, more preferably 1 minute or more, and preferably 3 minutes or more, and more preferably 5 minutes or more, and 60 minutes or less, preferably 45 minutes or less, and more preferably 30 minutes or less, from the viewpoint of improving the spiral rate, uniformity of the curl, and detanglability of the curl of the hair after treatment. When standing is performed while heating with a heater and the like as described above, the standing time can be further shortened, and the standing time

in this case is 15 seconds or more, preferably 30 seconds or more, more preferably 1 minute or more, preferably 3 minutes or more, and more preferably 5 minutes or more, and preferably 30 minutes or less, more preferably 15 minutes or less, and further preferably 10 minutes or less.

In the present invention, the standing time in step [b1] means the time after the second hair treatment agent is applied to the hair and until the next step, i.e., until the step other than the standing step.

Typically, the penetration into the hair of a component is advantageously performed by standing the hair under heating conditions for a long period of time, but the present invention is excellent in terms of obtaining the advantageous effects of the invention even by standing at room temperature for a short time.

Step [b1] is preferably performed in an environment in which the evaporation of moisture is suppressed. Examples of specific means for suppressing the evaporation of moisture include a method for covering the hair to which the second hair treatment agent is applied in step [B] with a plastic film, a cap, or the like, a method for continuously spraying water vapor such as superheated water vapor to the hair.

The second hair treatment agent may be rinsed or may not be rinsed from the hair after step [b1] and before the next step (i.e., the above-described step [C], step [A-1], or step [A-2]). In the case where the hair is rinsed after step [b1] and before the next step, the hair may be dried or may not be dried after rinsing and before performing the next step. After rinsing and before performing the next step, the hair may be untangled by hands or with a tool such as a comb or a hairbrush, or may not be untangled. A step of washing the hair may be provided after step [b1] and before the next step.

[Step [C]: Step of Applying Third Hair Treatment Agent Containing Component (C) to the Hair]

In step [C], the amount of the third hair treatment agent applied to the hair is such that the bath ratio relative to the mass of the hair (mass of hair treatment agent/mass of hair) is preferably 0.05 or more, more preferably 0.1 or more, and further preferably 0.2 or more, and preferably 1.5 or less, more preferably 1.25 or less, and further preferably 1.0 or less, from the viewpoint of improving the spiral rate, uniformity of the curl, and detanglability of the curl of the hair after treatment after hair treatment. The hair to be treated may be the whole head hair or may be a portion thereof, but the third hair treatment agent is preferably applied to the portion where the second hair treatment agent is applied in step [B].

To spread the third hair treatment agent over the entire head hair after applying the third hair treatment agent to the hair, a method with hands such as massaging the hair and the agent, or hand combing, a method using a tool such as a brush, a comb, and a hairbrush, and a combination of both methods may be used.

The hair treatment method of the present invention includes not only the method for performing the step [A-1], step [A-2], step [B], and step [C] in the order of step [A-1] or step [A-2] → step [B] → step [C], or in the order of step [B] → step [A-1] or step [A-2] → step [C] without overlapping with each other, but also aspects in which two or three steps of step [A-1], step [B], and step [C] or two or three steps of step [A-2], step [B], and step [C] are partially simultaneously performed.

That is, for example, in the case of performing in the order of step [A-1] → step [B] → step [C], step [A-1] may be performed on a portion of the hair, and then, while performing step [B] on this portion, step [A-1] may be performed on a portion of the hair where step [A-1] is not performed. Thereafter, when step [A-1] is further performed on a portion of the hair where step [A-1] is not performed, step [C] is performed on the portion of the hair where step [B] is finished, and step [B] is performed on the portion of the hair where step [A-1] is finished. In this case, step [A-1], step [B], and step [C] are simultaneously performed. In summary, each step is only required to be performed for each section of the hair to be treated, in the order of step [A-1] or step [A-2] → step [B] → step [C], or in the order of step [B] → step [A-1] or step [A-2] → step [C] .

[Step [c1]: Step of Leaving Hair To Stand After Step [C]]

After step [C], the hair may be rinsed or may not be rinsed, but is preferably rinsed from the viewpoint of making the soft touch and detanglability of hair after treatment satisfactory. The present invention may also include a step of washing the hair, after step [C].

In the case where the hair is rinsed after step [C], further, the following step [c1] is preferably performed between step [C] and the step of rinsing the hair, from the viewpoint of sufficiently obtaining the effects of the present invention.

Step [c1]: A step of standing the hair to which the third hair treatment agent is applied in step [C], at 15°C or more and 100°C or less for 1 minute or more and 60 minutes or less

The temperature for standing in step [c1] is 15°C or more and 100°C or less, preferably 60°C or less, and more preferably 30°C or less. The temperature is preferably 15°C or more and less than 30°C, i.e., room temperature in terms of convenient treatment without a need for a special device in the hair treatment. On the other hand, from the viewpoint of further shortening the standing time, it is possible to stand while warming with a heater and the like, and the temperature

in this case is preferably 30°C or more, and more preferably 40°C or more, and 100°C or less, more preferably 60°C or less, and further preferably 45°C or less.

The time for leaving the hair to stand in step [c1] after the third hair treatment agent is applied in step [C] is 1 minute or more, preferably 3 minutes or more, and more preferably 5 minutes or more, and 60 minutes or less, preferably 45 minutes or less, and more preferably 30 minutes or less, from the viewpoint of imparting soft feel to the hair while alleviating a kinky hair, aligning hair streaks of the hair, and imparting manageability. When standing is performed while heating with a heater and the like, as described above, the time for leaving the hair to stand can be further shortened, and the time for leaving the hair to stand in this case is 1 minute or more, preferably 3 minutes or more, more preferably 5 minutes or more, and further preferably 10 minutes or more, and preferably 30 minutes or less, and more preferably 15 minutes or less.

In the present invention, the time for leaving the hair to stand in step [c1] means the time after the third hair treatment agent is applied to the hair in step [C] and until the next step, i.e., until the step other than the step to leaving the hair to stand.

Typically, the penetration into the hair of a component is advantageously performed by leaving the hair to stand under heating conditions for a long period of time, but the present invention is excellent in terms of obtaining the advantageous effects of the invention even by leaving the hair to stand at room temperature for a short time. In the hair treatment method of the present invention, it is inferred that applying the second hair treatment agent to the hair in advance in step [B] changes the characteristics of the hair and promotes the penetration into the hair of the component (C), whereby such effects can be obtained.

Step [c1] is preferably performed in an environment in which the evaporation of moisture is suppressed. Examples of specific means for suppressing the evaporation of moisture include a method for covering the hair to which the third hair treatment agent is applied with a plastic film, a cap, or the like, and a method for continuously spraying water vapor such as superheated water vapor to the hair.

In the case where a step of rinsing the hair is performed after step [c1], a step of washing the hair can be included thereafter. In the case where the step of washing the hair is included, a commercially available shampoo and the like can be used for washing. After washing the hair, a step of treating the hair with a commercially available conditioner, treatment, and the like can be included.

The hair may be dried or may not be dried after the step of rinsing the hair, but is more preferably dried from the viewpoint of preventing the damage to the hair. Drying may be performed by natural drying, heat drying with such as a drier, and the like, but heat drying is preferable from the viewpoint of shortening the procedure time. From the viewpoint of further improving manageability and preventing hair damage, the hair is preferably untangled appropriately with hands or a tool such as a comb and a hairbrush, before drying step, during drying step, and/or after drying step.

The hair treatment method of the present invention is a technique capable of aligning the curl phase of the hair according to a principle completely different from a permanent treatment using a reducing agent and a so-called relaxer treatment using a strong alkaline hair treatment agent, not including a step of applying any of a hair treatment agent containing a reducing agent and a hair treatment agent with a pH of from 12 to 14. Thus, the hair treatment method of the present invention can deform the hair without damaging the hair.

Regarding the aforementioned embodiments, a preferred aspect of the present invention is further disclosed below. A hair treatment method performing the following steps 1 to 6 in the order presented.

1. A step of applying the first hair treatment agent containing the following component (A) to hair, then washing the hair with a shampoo, and rinsing with water.

   (A): Succinic acid from 4.5 to 6% by mass

2. A step of removing the moisture by drying with a towel.
3. A step of applying from 40 to 80 g of the second hair treatment agent containing the following components (B), (D), (E), and (G) to the hair and spreading the agent.

   (B): Malic acid and/or lactic acid from 1 to 15% by mass
   (D): Glycylglycine and/or isostearyl glyceryl ether from 0.8 to 6% by mass
   (E): Phenoxyethanol and/or benzyl alcohol from 0.4 to 6% by mass
   (G): Ethanol from 12.5 to 22% by mass

4. A step of applying from 40 to 80 g of the third hair treatment agent containing the following components (C) and (E) to the hair over the portion where the second hair treatment agent is applied and spreading the agent.

   (C): Sodium naphthalene sulfonate and/or sodium xylene sulfonate from 2.5 to 10% by mass

(E): Benzyl alcohol from 2 to 10% by mass

5. A step of warming the hair to which the third hair treatment agent is applied to from 40 to 45°C and standing the hair for from 10 to 30 minutes.
6. A step of rinsing the hair with water.

Examples

Examples 1 to 13 and Comparative Examples 1 to 5

The first to third hair cosmetics shown in Tables 2 to 5 were used to treat curly hair according to the following procedure, and the spiral rate, tress width, and detanglability of the curly hair before and after treatment were evaluated according to the following methods. These results are also shown in Tables 2 to 5.

(Tress for Evaluation)

Caucasian curly hair with no chemical treatment history (purchased from Kerling Corp.) was used to produce a tress having a length of 15 cm when the hair was pulled and straightened and a weight of 0.25 g.

(Hair Treatment Procedure)

a-1. After 0.5 g of a hair treatment agent for step [A-1] (first hair treatment agent) was applied to the tress for evaluation, it was uniformly spread over the hair and then allowed to stand, and thereafter, the hair was rinsed with tap water at 40°C for 30 seconds and dried with a towel.
a-2. After 0.25 g of a hair treatment agent for step [B] (second hair treatment agent) was applied, it was uniformly spread over the hair, and then the entire hair was covered with plastic wrap, sealed, and allowed to stand still for 5 minutes while warming to 40°C.
a-3. The plastic wrap was removed, and without rinsing the tress, 0.25 g of a hair treatment agent for step [C] (the third hair treatment agent) was further applied and spread, and then, the entire tress was covered with plastic wrap again and allowed to stand still at room temperature for 10 minutes.
a-4. The plastic wrap was removed and rinsed with tap water at 40°C for 30 seconds.
a-5. The tress after treatment (in a state of being rinsed with tap water at 40°C for 30 seconds) was immersed in 60 g of ethanol for 10 seconds.
a-6. The tress of the procedure 5 was fixed to a stirrer equipped with plate blades (manufactured by IKA-Werka), and rotated at 150 rpm for 18 minutes for drying.

<Spiral Rate of Curly Hair>

(Calculation Method for Spiral Rate Immediately After Treatment)

b-1. In the tress after drying in the treatment procedure a-6, the number of curling portions was counted from the fixed portion to the tips of the hair and taken as the total number of curls.
b-2. The tress was slowly placed on a towel from the tips of the hair such that the tress was perpendicular to the towel, in order to distinguish between curls having an aligned curl direction and in a spiral shape were discriminated and curls having a reversed curl direction and not having a spiral shape.
b-3. The number of curls in a spiral shape was counted based on the discrimination result of the procedure b-2, and the spiral rate of the curly hair was calculated according to the following formula.

$$\text{Spiral rate (\%)} = \text{number of spiral curls}/\text{total number of curls} \times 100$$

(Evaluation Method for Resistance to Shampooing)

c-1. The tress of the treatment procedure a-4 was lathered with a commercially available shampoo for 30 seconds, and rinsed with tap water at 40°C for 30 seconds.
c-2. The hair was dried according to the same step as the treatment procedure a-5 to a-6, and the spiral rate was calculated in the same manner as the b-1 to b-3.
c-3. The above steps of c-1 and c-2 were repeated 10 times, whereby the spiral rate was calculated. Three tresses were used for each evaluation, and the average value thereof was calculated, the value was used for evaluation of the resistance to shampooing with respect to the spiral rate of the curly hair.

<Tress Width of Curly Hair>

(Calculation Method for Tress Width Immediately After Treatment)

d-1. In the tress after drying in the treatment procedure a-6, the tress widths of arbitrary three points located in the middle of adjacent two curl portions were counted.
d-2. The tress widths of arbitrary three points in three tresses in total were counted in the same manner, and an average value of the tress widths of nine points in total was calculated, which was taken as the tress width of the curly hair immediately after treatment.

(Evaluation Method for Resistance to Shampooing)

e-1. The tress of the treatment procedure a-4 was lathered with a commercially available shampoo for 30 seconds, and rinsed with tap water at 40°C for 30 seconds.
e-2. The hair was dried according to the same step as the treatment procedure a-5 to a-6, and the tress width of the hair was calculated in the same manner as the d-1 to d-2.
e-3. The above steps of e-1 and e-2 were repeated 10 times, whereby the average value was evaluated as the resistance to shampooing with respect to the tress width of the curly hair.

<Detanglability of Curly Hair>

(Evaluation Method for Detanglability Immediately After Treatment)

f-1. For the tress after drying of the treatment procedure a-6, the detanglability of the hair was evaluated using the hair treated with the model treatment produced as follows as the standard hair, and seven panelists selected one of "less tangled than the standard hair" / "neither" / "more tangled than the standard hair".
f-2. The number of panelists who answered "less tangled than the standard hair" / "neither" / "more tangled than the standard hair" was counted, and evaluated as the detanglability of the curly hair immediately after treatment.

(Evaluation Method for Resistance to Shampooing)

g-1. The tress of the treatment procedure a-4 was lathered with a commercially available shampoo for 30 seconds, and rinsed with tap water at 40°C for 30 seconds.
g-2. The hair was dried according to the same step as the treatment procedure a-5 to a-6, and the detanglability of the curly hair was evaluated in the same manner as the f-1 to f-2.
g-3. The above steps of g-1 and g-2 were repeated 10 times, and the average value was evaluated as the resistance to shampooing with respect to the detanglability of the curly hair.

(Production Method for Hair Treated with Model Treatment)

h-1. After the tress after drying in the treatment procedure a-6 was washed with a commercially available shampoo, 1 g of a model treatment having the formulation shown in Table 1 was applied.
h-2. After standing for 30 minutes, the tress was rinsed with tap water at 40°C for 30 seconds, and then the hair was dried in the same procedure as the treatment procedure a-5 to a-6 to give the hair treated with the model treatment.

[Table 1]

| Formulation of model treatment | % by mass |
|---|---|
| Stearyl alcohol | 6.9 |
| Stearoxypropyldimethylamine (*1) | 2.0 |
| Sunflower seed oil | 1.5 |
| Hydrogenated castor oil hydroxystearate (*2) | 0.1 |
| Dipentaerythrityl hexahydroxystearate/hexastearate/hexarosinate (*3) | 0.4 |
| Lanolin acid | 0.18 |
| Dimethicone (*4) | 3.2 |
| Amodimethicone (*5) | 0.3 |
| Bis-isobutyl PEG-15/amodimethicone copolymer | 0.08 |
| Lactic acid | 1.3 |
| Dipropylene glycol | 3.5 |
| Benzyl alcohol | 0.4 |
| Fragrance | 0.45 |
| Ion-exchange water | Balance |
| * 1: FARMIN DM E-80 (manufactured by Kao Corporation)<br>*2: Technol MH (manufactured by Yokozeki Oil & Fat Industries Co., Ltd.)<br>*3: COSMOL 168ARV (manufactured by The Nisshin OilliO Group, Ltd.)<br>*4: Silicone KHS-3 (manufactured by Shin-Etsu Chemical Co., Ltd.)<br>*5: Silicone KT0032 (manufactured by Momentive Performance Materials Japan LLC) | |

[Table 2]

<table>
<tr><td colspan="3" rowspan="2">(All contents are expressed in active amount; % by mass)</td><td>Example</td><td colspan="3">Comparative Examples</td></tr>
<tr><td>1</td><td colspan="2">1 2</td><td>3</td></tr>
<tr><td rowspan="5">First hair treatment agent</td><td>(A1)</td><td>Succinic acid</td><td>5</td><td>-</td><td>5</td><td>5</td></tr>
<tr><td></td><td>Sodium hydroxide</td><td>(*6)</td><td>(*6)</td><td>(*6)</td><td>(*6)</td></tr>
<tr><td></td><td>Water</td><td>Balance</td><td>Balance</td><td>Balance</td><td>Balance</td></tr>
<tr><td colspan="2">Total</td><td>100</td><td>100</td><td>100</td><td>100</td></tr>
<tr><td colspan="2">pH</td><td>3.6</td><td>3.6</td><td>3.6</td><td>3.6</td></tr>
</table>

(continued)

| (All contents are expressed in active amount; % by mass) | | | Example | Comparative Examples | | |
|---|---|---|---|---|---|---|
| | | | 1 | 1 | 2 | 3 |
| Second hair treatment agent | (B) | Malic acid | 1.5 | 1.5 | - | - |
| | | Lactic acid | 3.5 | 3.5 | - | - |
| | (D) | Isostearyl glyceryl ether (*7) | 1 | 1 | - | - |
| | | Glycylglycine | 1 | 1 | - | - |
| | (E) | Benzyl alcohol | 4 | 4 | - | - |
| | (G) | Ethanol | 20 | 20 | - | - |
| | | Sodium hydroxide | (*6) | (*6) | (*6) | (*6) |
| | | Water | Balance | Balance | Balance | Balance |
| | Total | | 100 | 100 | 100 | 100 |
| | pH | | 3.6 | 3.6 | 3.6 | 3.6 |
| Third hair treatment agent | (C) | Sodium naphthalene sulfonate | 4 | 4 | 4 | - |
| | | Sodium xylene sulfonate | 5 | 5 | 5 | - |
| | (E) | Benzyl alcohol | 4 | 4 | 4 | - |
| | | Potassium phosphate, dipotassium phosphate | (*6) | (*6) | (*6) | (*6) |
| | | Water | Balance | Balance | Balance | 100 |
| | Total | | 100 | 100 | 100 | 100 |
| | pH | | 6.8 | 6.8 | 6.8 | 6.8 |
| Evaluation item | Spiral rate of curly hair (immediately after treatment) | | 83 | 80 | 78 | 43 |
| | Spiral rate of curly hair (resistance to shampooing) | | 80 | 40 | 41 | 38 |
| | Bundle width of curly hair (immediately after treatment) | | 0.55 | 0.52 | 0.57 | 0.76 |
| | Bundle width of curly hair (resistance to shampooing) | | 0.53 | 0.59 | 0.82 | 1.09 |
| | Detanglability of curly hair (immediately after treatment) | | 7/0/0 | 6/1/0 | 4/3/0 | 2/5/0 |
| | Detanglability of curly hair (resistance to shampooing) | | 6/1/0 | 4/2/1 | 2/2/3 | 1/1/5 |

*6: Amount of pH adjustment
*7: PENETOL GE-IS (manufactured by Kao Corporation)

[Table 3]

| (All contents are expressed in active amount; % by mass) | | | Examples | | | | Comparative Examples | |
|---|---|---|---|---|---|---|---|---|
| | | | 2 | 3 | 4 | 5 | 4 | 5 |
| First hair treatment agent | (A1) | Succinic acid | 5 | - | - | - | - | - |
| | | Tartaric acid | - | 5 | - | - | - | - |
| | | Mandelic acid | - | - | 5 | - | - | - |
| | | Phenyllactic acid | - | - | - | 5 | - | - |
| | (A') | Citric acid | - | - | - | - | 5 | - |
| | | EDTA | - | - | - | - | - | 5 |
| | | Sodium hydroxide | (*6) | (*6) | (*6) | (*6) | (*6) | (*6) |
| | | Water | Balance | Balance | Balance | Balance | Balance | Balance |
| | Total | | 100 | 100 | 100 | 100 | 100 | 100 |
| | pH | | 3.6 | 3.6 | 3.6 | 3.6 | 3.6 | 3.6 |
| Second hair treatment agent | (B) | Malic acid | 1.5 | 1.5 | 1.5 | 1.5 | 1.5 | 1.5 |
| | | Lactic acid | 3.5 | 3.5 | 3.5 | 3.5 | 3.5 | 3.5 |
| | (D) | Isostearyl glyceryl ether (*7) | 1 | 1 | 1 | 1 | 1 | 1 |
| | | Glycylglycine | 1 | 1 | 1 | 1 | 1 | 1 |
| | (E) | Benzyl alcohol | 4 | 4 | 4 | 4 | 4 | 4 |
| | (G) | Ethanol | 20 | 20 | 20 | 20 | 20 | 20 |
| | | Sodium hydroxide | (*6) | (*6) | (*6) | (*6) | (*6) | (*6) |
| | | Water | Balance | Balance | Balance | Balance | Balance | Balance |
| | Total | | 100 | 100 | 100 | 100 | 100 | 100 |
| | pH | | 3.6 | 3.6 | 3.6 | 3.6 | 7 | 7 |
| Third hair treatment agent | (C) | Sodium naphthalene sulfonate | 4 | 4 | 4 | 4 | 4 | 4 |
| | | Sodium xylene sulfonate | 5 | 5 | 5 | 5 | 5 | 5 |
| | (E) | Benzyl alcohol | 4 | 4 | 4 | 4 | 4 | 4 |
| | | Potassium phosphate, dipotassium phosphate | (*6) | (*6) | (*6) | (*6) | (*6) | (*6) |
| | | Water | Balance | Balance | Balance | Balance | Balance | Balance |
| | Total | | 100 | 100 | 100 | 100 | 100 | 100 |
| | pH | | 6.8 | 6.8 | 6.8 | 6.8 | 6.8 | 6.8 |

(continued)

| (All contents are expressed in active amount; % by mass) | | Examples | | | | Comparative Examples | |
|---|---|---|---|---|---|---|---|
| | | 2 | 3 | 4 | 5 | 4 | 5 |
| Evaluation item | Spiral rate of curly hair (immediately after treatment) | 83 | 82 | 82 | 82 | 50 | 52 |
| | Spiral rate of curly hair (resistance to shampooing) | 80 | 75 | 77 | 75 | 38 | 38 |
| | Bundle width of curly hair (immediately after treatment) | 0.55 | 0.52 | 0.54 | 0.55 | 0.81 | 0.74 |
| | Bundle width of curly hair (resistance to shampooing) | 0.53 | 0.55 | 0.55 | 0.52 | 1.12 | 1.09 |
| | Detanglability of curly hair (immediately after treatment) | 7/0/0 | 5/1/1 | 6/1/0 | 6/1/0 | 2/2/3 | 1/3/3 |
| | Detanglability of curly hair (resistance to shampooing) | 6/1/0 | 5/1/1 | 5/1/1 | 5/1/1 | 1/1/5 | 1/1/5 |
| *6: Amount of pH adjustment<br>*7: PENETOL GE-IS (manufactured by Kao Corporation) | | | | | | | |

[Table 4]

| (All contents are expressed in active amount; % by mass) | | | Examples | | |
|---|---|---|---|---|---|
| | | | 6 | 7 | 8 |
| First hair treatment agent | (A1) | Succinic acid | 10 | 2 | 1 |
| | | Tartaric acid | - | - | - |
| | | Mandelic acid | - | - | - |
| | | Phenyllactic acid | - | - | - |
| | (A') | Citric acid | - | - | - |
| | | EDTA | - | - | - |
| | | Sodium hydroxide | (*6) | (*6) | (*6) |
| | | Water | Balance | Balance | Balance |
| | Total | | 100 | 100 | 100 |
| | pH | | 3.6 | 3.6 | 3.6 |
| Second hair treatment agent | (B) | Malic acid | 1.5 | 1.5 | 1.5 |
| | | Lactic acid | 3.5 | 3.5 | 3.5 |
| | (D) | Isostearyl glyceryl ether (*7) | 1 | 1 | 1 |
| | | Glycylglycine | 1 | 1 | 1 |
| | (E) | Benzyl alcohol | 4 | 4 | 4 |
| | (G) | Ethanol | 20 | 20 | 20 |
| | | Sodium hydroxide | (*6) | (*6) | (*6) |
| | | Water | Balance | Balance | Balance |
| | Total | | 100 | 100 | 100 |
| | pH | | 3.6 | 7 | 3 |

(continued)

| (All contents are expressed in active amount; % by mass) | | | Examples | | |
|---|---|---|---|---|---|
| | | | 6 | 7 | 8 |
| Third hair treatment agent | (C) | Sodium naphthalene sulfonate | 4 | 4 | 4 |
| | | Sodium xylene sulfonate | 5 | 5 | 5 |
| | (E) | Benzyl alcohol | 4 | 4 | 4 |
| | | Potassium phosphate, dipotassium phosphate | (*6) | (*6) | (*6) |
| | | Water | Balance | Balance | Balance |
| | Total | | 100 | 100 | 100 |
| | pH | | 6.8 | 6.8 | 6.8 |
| Evaluation item | Spiral rate of curly hair (immediately after treatment) | | 85 | 80 | 75 |
| | Spiral rate of curly hair (resistance to shampooing) | | 83 | 70 | 65 |
| | Bundle width of curly hair (immediately after treatment) | | 0.52 | 0.53 | 0.52 |
| | Bundle width of curly hair (resistance to shampooing) | | 0.51 | 0.60 | 0.62 |
| | Detanglability of curly hair (immediately after treatment) | | 7/0/0 | 7/0/0 | 6/1/0 |
| | Detanglability of curly hair (resistance to shampooing) | | 7/0/0 | 7/0/0 | 6/1/0 |

*6: Amount of pH adjustment
*7: PENETOL GE-IS (manufactured by Kao Corporation)

[Table 5]

| (All contents are expressed in active amount; % by mass) | | | Examples | | | | |
|---|---|---|---|---|---|---|---|
| | | | 9 | 10 | 11 | 12 | 13 |
| First hair treatment agent | (A1) | Succinic acid | 5 | 5 | 5 | 5 | 5 |
| | | Sodium hydroxide | (*6) | (*6) | (*6) | (*6) | (*6) |
| | | Water | Balance | Balance | Balance | Balance | Balance |
| | Total | | 100 | 100 | 100 | 100 | 100 |
| | pH | | 3.6 | 3.6 | 3.6 | 3.6 | 3.6 |
| Second hair treatment agent | (B) | Malic acid | 5 | - | 1.5 | 1.5 | 1.5 |
| | | Lactic acid | - | 5 | 3.5 | 3.5 | 3.5 |
| | (D) | Isostearyl glyceryl ether (*7) | 1 | 1 | - | 1 | - |
| | | Glycylglycine | 1 | 1 | 1 | - | - |
| | (E) | Benzyl alcohol | 4 | 4 | 4 | 4 | 4 |
| | (G) | Ethanol | 20 | 20 | 20 | 20 | 20 |
| | | Sodium hydroxide | (*6) | (*6) | (*6) | (*6) | (*6) |
| | | Water | Balance | Balance | Balance | Balance | Balance |
| | Total | | 100 | 100 | 100 | 100 | 100 |
| | pH | | 3.6 | 3.6 | 3.6 | 3.6 | 3.6 |

(continued)

| (All contents are expressed in active amount; % by mass) | | | Examples | | | | |
|---|---|---|---|---|---|---|---|
| | | | 9 | 10 | 11 | 12 | 13 |
| Third hair treatment agent | (C) | Sodium naphthalene sulfonate | 4 | 4 | 4 | 4 | 4 |
| | | Sodium xylene sulfonate | 5 | 5 | 5 | 5 | 5 |
| | (E) | Benzyl alcohol | 4 | 4 | 4 | 4 | 4 |
| | | Potassium phosphate, dipotassium phosphate | (*6) | (*6) | (*6) | (*6) | (*6) |
| | | Water | Balance | Balance | Balance | Balance | Balance |
| | Total | | 100 | 100 | 100 | 100 | 100 |
| | pH | | 6.8 | 6.8 | 6.8 | 6.8 | 6.8 |
| Evaluation item | Spiral rate of curly hair (immediately after treatment) | | 83 | 77 | 82 | 82 | 82 |
| | Spiral rate of curly hair (resistance to shampooing) | | 79 | 70 | 72 | 71 | 65 |
| | Bundle width of curly hair (immediately after treatment) | | 0.51 | 0.61 | 0.52 | 0.53 | 0.53 |
| | Bundle width of curly hair (resistance to shampooing) | | 0.53 | 0.64 | 0.62 | 0.63 | 0.72 |
| | Detanglability of curly hair (immediately after treatment) | | 6/1/0 | 6/1/0 | 6/1/0 | 6/1/0 | 5/2/0 |
| | Detanglability of curly hair (resistance to shampooing) | | 6/1/0 | 5/2/0 | 4/3/0 | 5/2/0 | 4/2/1 |
| *1: Amount of pH adjustment<br>*2: PENETOL GE-IS (manufactured by Kao Corporation) | | | | | | | |

**Claims**

**1.** A hair treatment method comprising the following steps [A-1], [B], and [C], wherein the method is carried out in the order of step [A-1] → step [B] → step [C], or in the order of step [B] → step [A-1] → step [C]:

step [A-1]: a step of applying a first hair treatment agent comprising an organic acid having a solubility in 100 mL of water at 20°C of 50 g or less or a salt of the organic acid, as component (A1), to hair, and rinsing the hair;
step [B]: a step of applying a second hair treatment agent comprising a carboxylic acid having an inorganic value of 250 or more and 450 or less and an organic value of 50 or more and 250 or less or a salt of the carboxylic acid, as component (B), to the hair; and
step [C]: a step of applying a third hair treatment agent comprising an aromatic sulfonic acid having a molecular weight of 300 or less or a salt thereof, as component (C), to the hair.

**2.** A hair treatment method comprising the following steps [A-2], [B], and [C], wherein the method is carried out in the order of step [A-2] → step [B] → step [C], or in the order of step [B] → step [A-2] → step [C]:

step [A-2]: a step of applying a first hair treatment agent comprising an organic acid being more hydrophobic than a carboxylic acid of component (B) or a salt of the organic acid, as component (A2), to hair;
step [B]: a step of applying a second hair treatment agent comprising a carboxylic acid having an inorganic value of 250 or more and 450 or less and an organic value of 50 or more and 250 or less or a salt of the carboxylic acid, as the component (B), to the hair; and

step [C]: a step of applying a third hair treatment agent comprising an aromatic sulfonic acid having a molecular weight of 300 or less or a salt thereof, as component (C), to the hair.

3. The hair treatment method according to claim 1 or 2, wherein step [b1] of leaving the hair to which the second hair treatment agent is applied to stand at 15°C or more and 100°C or less for 15 seconds or more and 60 minutes or less is further performed subsequent to step [B].

4. The hair treatment method according to any one of claims 1 to 3, wherein the component (A1) or the component (A2) is at least one selected from the group consisting of succinic acid and tartaric acid.

5. The hair treatment method according to any one of claims 1 to 4, wherein the pH of the first hair treatment agent at 25°C is 2 or more and 6.5 or less.

6. The hair treatment method according to any one of claims 1 to 5, wherein the second hair treatment agent further comprises at least one compound selected from the group consisting of a glycylglycine derivative of formula (1) or a salt thereof, a polyglycerin having an average degree of polymerization of 2 or more and 6 or less, and an alkyl glyceryl ether in which the alkyl group has 6 or more and 40 or less carbon atoms, as component (D):

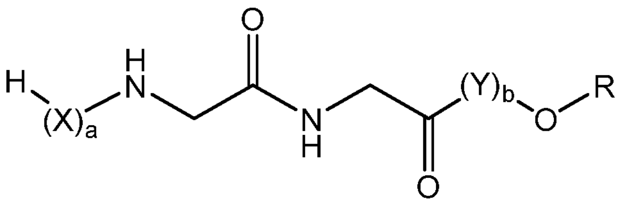

(1)

wherein X represents an optionally hydroxy group-substituted divalent hydrocarbon group having 1 to 4 carbon atoms, or an amino acid residue;
Y represents an amino acid residue, or a divalent group represented by Formula (2):

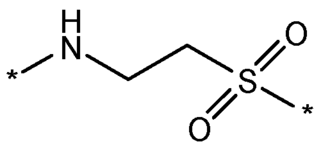

(2)

wherein, -* represents a bond which bonds to an adjacent carbonyl group or oxygen atom;

R represents a hydrogen atom or an optionally hydroxy group-substituted monovalent hydrocarbon group having 1 to 4 carbon atoms; and
a and b represent 0 or 1, provided that, when a and b are simultaneously 1, X is not an amino acid residue.

7. The hair treatment method according to any one of claims 1 to 6, comprising a step of rinsing the hair after step [C], and further comprising step [c1] between step [C] and the step of rinsing the hair:
step [c1]: a step of leaving the hair to which the third hair treatment agent is applied to stand at 15°C or more and 100°C or less for 1 minute or more and 60 minutes or less.

8. The hair treatment method according to any one of claims 1 to 7, wherein the content of (E) an aromatic alcohol in the second hair treatment agent is 0.5% by mass or more and 15% by mass or less.

9. The hair treatment method according to any one of claims 1 to 8, wherein the second hair treatment agent further comprises a thickener as component (F).

10. The hair treatment method according to any one of claims 1 to 9, wherein the pH of the first hair treatment agent at 25°C is 2 or more and 10 or less.

11. The hair treatment method according to any one of claims 1 to 10, wherein the pH of the second hair treatment agent at 25°C is 2 or more and 10 or less.

**12.** The hair treatment method according to any one of claims 1 to 11, wherein the third hair treatment agent further comprises a thickener as the component (F), and an aromatic alcohol as the component (E), and the content of the component (F) is from 0.1% by mass to 5% by mass.

**13.** The hair treatment method according to any one of claims 7 to 12, wherein the temperature for leaving the hair to stand in step [c1] is 15°C or more and less than 30°C.

**14.** The hair treatment method according to any one of claims 7 to 13, wherein the temperature for leaving the hair to stand in step [c1] is 30°C or more and 100°C or less, and the standing time is 1 minute or more and 30 minutes or less.

**15.** The hair treatment method according to any one of claims 1 to 14, not comprising a step of applying any of a hair treatment agent containing a reducing agent and a hair treatment agent with a pH of from 12 to 14.

**16.** A hair treatment method comprising the following steps [A-1], [B], and [C], wherein the method is carried out in the order of step [A-1] → step [B] → step [C], or in the order of step [B] → step [A-1] → step [C]:

step [A-1]: a step of applying 0.5% by mass or more and 15% by mass or less of a first hair treatment agent comprising at least one organic acid selected from the group consisting of succinic acid and tartaric acid or a salt of the organic acid, as component (A1), to hair, and rinsing the hair;
step [B]: a step of applying a second hair treatment agent comprising 1% by mass or more and 25% by mass or less of malic acid, lactic acid, or salts thereof as component (B), and 0.5% by mass or more and 10% by mass or less of at least one compound selected from the group consisting of a glycylglycine derivative of formula (1) or a salt thereof, a polyglycerin having an average degree of polymerization of 2 or more and 6 or less, and an alkyl glyceryl ether in which the alkyl group has 6 or more and 40 or less carbon atoms, as component (D), to the hair; and
step [C]: a step of applying a third hair treatment agent comprising 0.4% by mass or more and 15% by mass or less of an aromatic sulfonic acid having a molecular weight of 300 or less or a salt thereof, as component (C), to the hair.

## INTERNATIONAL SEARCH REPORT

International application No.
PCT/JP2020/018109

A. CLASSIFICATION OF SUBJECT MATTER
Int. Cl. A61Q5/00(2006.01)i, A61Q5/04(2006.01)i, A61Q5/06(2006.01)i, A61Q5/12(2006.01)i, A61K8/362(2006.01)i, A61K8/365(2006.01)i, A61K8/46(2006.01)i
FI: A61K8/362, A61K8/46, A61K8/365, A61Q5/00, A61Q5/04, A61Q5/06, A61Q5/12

According to International Patent Classification (IPC) or to both national classification and IPC

B. FIELDS SEARCHED

Minimum documentation searched (classification system followed by classification symbols)
Int. Cl. A61Q5/00, A61Q5/04, A61Q5/06, A61Q5/12, A61K8/362, A61K8/365, A61K8/46

Documentation searched other than minimum documentation to the extent that such documents are included in the fields searched
Published examined utility model applications of Japan 1922-1996
Published unexamined utility model applications of Japan 1971-2020
Registered utility model specifications of Japan 1996-2020
Published registered utility model applications of Japan 1994-2020

Electronic data base consulted during the international search (name of data base and, where practicable, search terms used)
Mintel GNPD

C. DOCUMENTS CONSIDERED TO BE RELEVANT

| Category* | Citation of document, with indication, where appropriate, of the relevant passages | Relevant to claim No. |
|---|---|---|
| A | WO 2010/018668 A1 (KAO CORP.) 18 February 2010, entire text | 1-16 |
| A | JP 2007-176826 A (LION CORP.) 12 July 2007, entire text | 1-16 |
| A | JP 2009-537620 A (L'OREAL) 29 October 2009, entire text | 1-16 |
| A | JP 2016-530305 A (KAO CORP.) 29 September 2016, entire text | 1-16 |

☐ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16.06.2020 | 23.06.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/018109

| Patent Documents referred to in the Report | Publication Date | Patent Family | Publication Date |
|---|---|---|---|
| WO 2010/018668 A1 | 18.02.2010 | JP 2010-65022 A<br>US 2011/0150804 A1<br>entire text<br>EP 2314278 A1 | |
| JP 2007-176826 A | 12.07.2007 | (Family: none) | |
| JP 2009-537620 A | 29.10.2009 | US 2010/0300471 A1<br>entire text<br>WO 2007/135299 A1<br>EP 2029237 A1<br>FR 2901471 A1 | |
| JP 2016-530305 A | 29.09.2016 | US 2016/0228342 A1<br>entire text<br>WO 2015/036051 A1<br>EP 3046534 A1<br>CN 105555253 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **YAMORI.** Formulation Design with Organic Conception Diagram. *Fragrance Journal,* 1989, vol. 4, 29-38